# EUROPEAN PATENT APPLICATION

(11) **EP 1 193 311 A1**
(43) Date of publication of application: **03.04.2002**
(21) Application number: 01121265.1
(22) Date of filing: 05.09.2001
(51) Int. Cl.: C12N 15/10, C12N 1/19

(54) **Method for stimulating the mating of microorganisms in liquid medium**

(30) Priority: 27.09.2000 DE 10047951
(71) Applicant: LION Bioscience AG, 69120 Heidelberg (DE)
(72) Inventor: Albers, Michael, Dr., 69221 Dossenheim (DE); Kober, Ingo, Dr., 69251 Gaiberg (DE); Kögl, Manfred, Dr., 69214 Eppelheim (DE); Löser, Eva, 69121 Heidelberg (DE)
(74) Representative: Schüssler, Andrea, Dr.

(57) **Abstract**

The invention relates to a method of stimulating the mating of microorganisms with sexual or parasexual life cycle, preferably yeast (e.g. *Saccharomyces cerevisiae*) in a liquid medium, which is characterized in that the microorganisms of differing mating type are cultured in the liquid medium in the presence of a high-molecular swellable compound, preferably polyethylene glycol. The invention also relates to a screening method based thereon ("yeast two hybrid" system; Y2H).

## Description

The present invention relates to a method of stimulating the mating of microorganisms with sexual or parasexual life cycle, preferably yeast, e.g. *Saccharomyces cerevisiae,* in a liquid medium, which is characterized in that the microorganisms of differing mating type are cultured in the liquid medium in the presence of a high-molecular, swellable compound, preferably polyethylene glycol. The present invention also relates to a screening method based thereon ("yeast two hybrid" system; Y2H).

In the "yeast two hybrid" system (Y2H system) which is used *inter alia* in the field of screening for desired substances, two plasmids are placed jointly in a yeast cell to test whether the two fusion proteins expressed by these plasmids bind to each other. Binding results in the activation of reporter genes, which leads to a measurable phenotypic modification of the yeast cells. As a result of this modification the cells may grow on agar plates which contain no histidine, for example. In one of the common applications, a "bait" protein is tested for interaction with a large number of proteins from a gene library. For this purpose, a yeast strain which expresses the bait protein is contacted with the different clones from the gene library. This can be made in different ways, namely
(a) by transforming the yeast strain expressing the bait with the DNA of the gene library; or
(b) mating yeast cells. For this, a gene library pretransformed in yeast is used. This form of gene library consists of yeast cells carrying the different clones from the gene library and expressing the corresponding proteins. If a haploid yeast culture which expresses the bait protein is mixed with a haploid culture carrying the gene library, the yeast cells mate and fuse to form diploid cells. The precondition is that the two strains do not have the same mating type.

However, when gene libraries are analyzed, it is important to be able to test the largest possible number of clones to also comprise rare clones in the analysis. This is even more important when many different libraries shall be tested with a large number of baits. The methods common in the art thus far differ here as regards their usability.

The transformation of e.g. yeast cells comprises a number of incubations in different media and is concluded by plating the yeast cells on selective agar plates. This method is not suitable due to its relative complexity for screening on a relatively large scale. It is used above all where only few screenings are made and the expensive and time-consuming pretransformation of the libraries does not pay. Since the mating of yeast cells is in principle a simple process, this method is chosen when a large number of screenings shall be carried out. The critical factor is here the mating efficiency. The larger the percentage of cells fusing to form cygotes is, the more material can be screened with the same expenditure. Since only a limited number of yeast cells can be spread over a selective agar plate altogether, large screenings often require many hundred plates. In fact, handling the plates is the limiting factor in the process of screening, preventing that the method can even be extended. This applies similarly to other selection methods, such as sorting for fluorescence (FACS), sorting for binding to magnetic particles (MAGS) or the selection in liquid media.

There are various methods of increasing the amount of diploid cells in the mating process. All of the most efficient methods comprise a step in which the yeast cells are contacted on a solid support, such as a filter paper or an agar plate. Following incubation, the yeasts are washed off the support again and further processed. Although these methods achieve a basically good efficiency, they have the decisive drawback that the cells must first be applied onto the support and thereafter be flushed off again, which is a time-consuming process. This process cannot be automated either, which is opposed to a further acceleration of the method. Although a method in which the cells are mated in liquid medium is much simpler, the mating efficiency achievable by this is, however, very low, above all as regards screening on a large scale.

Thus, the present invention is based on the technical problem of overcoming these drawbacks of the methods used in the art, i.e. a method shall be provided which effects the mating of microorganisms with high mating efficiency and is simultaneously simple and cost-effective.

This technical problem is solved by providing the embodiments characterized in the claims. It was found surprisingly that microorganisms can also be mated with high efficiency in liquid medium when this medium contains a high-molecular swellable compound. This method is very well suited for efficient screening with high throughput since it allows to achieve efficient mating of the cells by simply mixing two yeast strains in liquid medium. In this connection, the addition of the above-mentioned high-molecular, swellable compound of the liquid medium, e.g. polyethylene glycol (PEG), causes aggregation of the cells for example, i.e. the cells are then available as small clots. It is assumed that this state is obviously similar to the state on the agar plate or a filter, i.e. results in increased mating efficiency. However, the steps of plating and flushing are omitted in the method according to the invention, which strongly accelerates the process. Since it is not necessary to use agar plates, the costs of the material and the expenditure required for casting the plates are saved. The incubation in this medium can be scaled without an upward limit as regards the quantity so that this step of the method is no longer the limiting factor.

Thus, the present invention relates to a method of stimulating the mating of microorganisms with sexual or parasexual life cycle in a liquid medium, characterized in that microorganisms of differing mating type are cultured in the liquid medium in the presence of a high-molecular swellable compound.

This compound causes the microorganisms to approach one another, flocculate or agglutinate, the growth properties of the microorganisms being preferably not affected negatively.

The liquid medium or basic medium, which is used for the method according to the invention may be any medium usually used for culturing microorganisms, preferably yeast cells, e.g. YPD, YPAD, SD, SCC or SMM (Adams *et al*., Methods in Yeast Genetics, Cold Spring Habour Laboratory Press, 1997, ISBN 0-87969-508-0). The further culturing conditions, e.g. temperature, O₂ supply, CO₂ supply, *etc.,* correspond to the conditions usually used for culturing the respective microorganisms and they are known to a person skilled in the art.

All microorganisms having a sexual or parasexual life cycle are suited for the method according to the invention, and they comprise *Escherichia coli* or *Bacillus subtilis,* for example.

In a preferred embodiment of the method according to the invention, the microorganisms are yeasts, e.g. klyveromyces, hansenula, saccharomyces, or schizosaccharomyces, with *Saccharomyces cerevisiae, Schizosaccharomyes pombe,* Klyveromyces lactis or Hansenula polymorpha being preferred and *Saccharomyces cerevisiae* or *Schizosaccharomyces pombe* being most preferred.

In principle, it is possible to use for the method according to the invention as an addition to the liquid medium any high-molecular swellable compound which dehydrates the medium and leads to a spatial approach, flocculation or agglutination of microorganisms in liquid media, e.g. compounds may be concerned such as agarose beads, sepharose® beads, cellulose powder, sephadex® , PEG, *etc*. The term "high-molecular" used herein preferably relates to a molecular weight range of 100 or more, preferably 1000 to 150,000 daltons, more preferably 3,000 to 100,000 daltons, and most preferably 5,000 to 50,000 daltons.

In a particularly preferred embodiment of the method according to the invention, the compound added to the liquid medium is a polyethylene glycol (PEG), the polyethylene glycol being preferably solid, i.e. it has a molecular weight of over 400 daltons. It is even more preferred to use a polyethylene glycol having a molecular weight ranging from 2,000 to 20,000, a polyethylene glycol having a molecular weight of 6,000 daltons being most preferred.

The concentration of the compound increasing the mating efficiency in the liquid medium is not critical, and a person skilled in the art can determine the most favorable concentration, i.e. the concentration resulting in the maximum mating efficiency without essentially impairing the growth or the survival rate of the cells by means of simple experiments, e.g. by means of the method described in the example below. The most favorable concentration also depends on the compound used in each case and the respective microorganism. In a preferred embodiments of the method according to the invention, the compound is present in the liquid medium in a concentration of 2 to 12 % by weight, a concentration of 10 % by weight being most preferred.

The optical density (OD₆₀₀), suitable for a maximum mating efficiency with otherwise least possible negative implications, of the microorganisms for the incubation in liquid medium and the duration of incubation are not critical either and can also be determined by the person skilled in the art by means of simple experiments, e.g. by means of the method described in the example below. The optimum optical density (OD₆₀₀) and the duration of incubation also depend on the compound used in each case and the respective microorganism. In a preferred embodiment of the method according to the invention, the microorganisms are incubated in the liquid medium with an OD₆₀₀ between 1.0 and 4.0, preferably for 3 to 4 hours.

In an even more preferred embodiment, the method according to the invention is further characterized in that
(a) the microorganisms of one mating type express a reporter protein and the microorganisms of the other mating type are transformed with a gene library,
(b) following incubation in the liquid medium the microorganism are isolated; and
(c) the microorganisms which show activation of the reporter are selected.

The person skilled in the art is familiar with methods of isolating microorganisms from liquid media, e.g. centrifugation. Methods of constructing gene libraries or recombinant vectors for expressing a reporter protein (or bait protein) are also known to the person skilled in the art. Suitable vectors, preferably expression vectors, are also known to the person skilled in the art. The vectors may be a plasmid or another suitable vehicle, for example. The vector is preferably functionally linked to regulatory elements permitting its expression in eukaryotic host cells. Preferred plasmids and vectors are: pAD-GAL4-2.1, pBD-GAL4, pBD-GAL4Cam, pCMV-AD, pCMV-BD, pMyr, pSos, pACT2, pAS2-1, pHISi, pLexA, pM, pHISi-1, pB42AD, pVP16, pGAD10, pGBKT7, pLacZi, p8op-lacZ, pGAD GH, pGilda, pAD GL, pGADT7, pGBDU, pDBLeu, pPC86, pDBTrp. Such vectors contain in addition to the regulatory elements, e.g. a promoter, typically a replication origin and specific genes which permit the phenotypic selection of a transformed host cell. The regulatory elements for the expression in yeast cells comprise the AOX1 or GAL1 promoter, ADH promoter, GAL1-10 promoter, Cup promoter or Met1 promoter. The vectors suitable for the expression in yeast comprise pY100 and Ycpad1 as well as the above-listed vectors, in particular those of the pRS series, the YCP or YEP series (Guthrie, C. and Fink, G. (editor), 1991, Methods in Enzymology 194, Guide to Yeast Genetics and Molecular Biology, Academic Press, San Diego, California 92101, ISBN 0-12-182095-5). Methods for the transformation of microorganisms and the phenotypic selection of transformants are known in the art or described sufficiently in the literature; see e.g. Sambrook *et al.,* Molecular Cloning: A Laboratory Manual, 2^{nd} edition, Cold Spring Harbor Laboratory Press, Cold Spring Harbor, N.Y. (1989).

The person skilled in the art is also familiar with suitable reporter proteins (or bait proteins) as a function of the screened-for substance from the gene library, which activates this reporter gene or the corresponding gene, and with corresponding selection methods. They comprise e.g. green, yellow or red fluorescent protein, β-galactosidase, α-galactosidase, β-lactamase, chloramphenical-acetyl transferase, β-glucuronidase, ADE2, ADE3, Leu2, His3, peroxidase, alkaline phosphatase, luciferase, surface antigens.

The following example explains the invention.

### Example

### Mating of yeast cells in liquid medium with 10 % PEG 6000

Deep-frozen yeast cells (strain CG-1945 or AH109, Mat*alpha*, Clontech yeast protocol handbook PT3024-1, Clontech company, Palo Alto, U.S.A.) of a Y2H library from human brain (order number HL4004AH, 1 ml; Clontech company, Palo Alto, U.S.A.) were thawed and incubated at 30°C for 1 hour in YPDA medium (10 g Bacto yeast extract, 20 g Bacto peptones, 20 g glucose, 40 mg adenine sulfate, water ad 1000 ml; sterilizing by autoclaving; ingredients from the company of DIFCO Laboratories, Detroit, U.S.A.). Cells of opposite mating type (MAT**a**) which expressed the ligand binding domain of the human PPARgamma protein in fusion with the DNA binding domain of the pGBKT7 vector (Clontech company, Palo Alto, U.S.A., catalog # K1612-B) (cell with bait or reporter protein) were grown overnight in selective medium to an OD₆₀₀ of 1.0. 20 O.D. units of cells of both yeast strains were mixed with one another, centrifuged off and resuspended in 15 ml YPDA medium. The OD₆₀₀ was measured and was 3.75. 7.5 ml of this mixture each were distributed over two Erlenmeyer flasks. 1.9 ml of a 50 % solution of PEG 6000 (company of Carl Roth, Karlsruhe, Germany) were added to one of the two flasks, so that the final concentration of PEG 6000 was about 10 %. Both cultures were incubated at 30°C while shaking (100 rpm) for four hours. Thereafter, the cells were centrifuged off, washed once in selective medium without leucine, histidine and tryptophan (Adams *et al.*, Methods in Yeast Genetics, Cold Spring Habour Laboratory Press, 1997, ISBN 0-87969-508-0) and resuspended in 30 ml of the same medium. The cells were diluted to a concentration of 1:500 in the same medium, and 50 µl were plated onto different plates (see Table 1). Following incubation at 30°C for 3 days, the number of colonies was determined. The result is shown in Table 1.

**Table 1**

| | Without PEG 6000 | 10 % PEG 6000 |
|---|---|---|
| -leucine (cells with the gene library) | 132 | 215 |
| -tryptophan (cells with the bait protein) | about 1000 | about 1000 |
| -leucine, -tryptophan (diploid cells) | 9 | 149 |

Table 1 shows that, following the correction to the same number of surviving cells from the library (plates - leucine), the quantity of diploid cells existing when 10 % PEG is present on plates without leucine and tryptophane (= selection for diploids) is ten times as much as that without PEG. Thus, about 22.35 million diploids formed in the 15 ml medium of the mating experiments carried out here when PEG is used for stimulating the mating, whereas only 1.35 million diploids were present without PEG.

## Claims

1. A method of stimulating the mating of microorganisms with sexual or parasexual life cycle in a liquid medium, **characterized in that** microorganisms of different mating type are cultured in the liquid medium in the presence of a high-molecular swellable compound.

2. The method according to claim 1, wherein the microorganism is a yeast.

3. The method according to claim 2, wherein the yeast is *Saccharomyces cerevisiae* or *Schizosaccharomyces pombe.*

4. The method according to any of claims 1 to 3, wherein the high-molecular swellable compound is polyethylene glycol.

5. The method according to claim 4, wherein polyethylene glycol has a molecular weight ranging from 2,000 to 20,000 daltons.

6. The method according to claim 5, wherein polyethylene glycol has a molecular weight of about 6,000 daltons.

7. The method according to any of claims 1 to 6, wherein the high-molecular swellable compound is present in a concentration ranging from 2 to 12 % by weight in the liquid medium.

8. The method according to claim 7, wherein the concentration is 10 % by weight.

9. The method according to any of claims 1 to 8, wherein the microorganisms in the liquid medium are incubated at an OD₆₀₀ between 1.0 and 4.0.

10. The method according to any of claims 1 to 9, further **characterized in that**
(a) the microorganisms of one mating type express a reporter protein and the microorganisms of the other mating type are transformed with a gene library;
(b) following incubation in the liquid medium the microorganisms are isolated; and
(c) the microorganisms which show activation of the reporter are selected.
